# EUROPEAN PATENT SPECIFICATION

(11) **EP 0 398 850 B1**
(45) Date of publication and mention of the grant of the patent: **15.03.1995**
(21) Application number: 90830215.1
(22) Date of filing: 16.05.1990
(51) Int. Cl.: A61L 11/00

(54) **A method and apparatus for the sterilization of waste materials of more in particular, special hospital waste materials**
Verfahren und Vorrichtung für die Sterilisierung von Abfallmaterialien, insbesondere von Krankenhausabfällen
Procédé et dispositif pour la stérilisation de déchets, notamment pour les déchets d'hôpitaux

(30) Priority: 19.05.1989 IT 4797289
(43) Date of publication of application: 22.11.1990
(73) Proprietor: RAPANELLI FIORAVANTE S.p.A., I-06034 Foligno(Perugia) (IT)
(72) Inventor: Rapanelli, Paolo, I-06034 Foligno (IT)
(74) Representative: Fiammenghi-Domenighetti, Delfina

(56) References cited:
- EP-A- 0 236 575
- Prof. Dr.-Ing. OTTO ANNA: "Müllsterilisation - Modell Medizinische Hochschule Hannover", March 1980

## Description

The present invention relates to a method and apparatus for sterilizing special waste materials, more in particular, hospital waste materials and the like coming from medication rooms, contagious disease departments, biological laboratories as well as any waste material that, however, can be detrimental to the public health.

According to the present law provisions, the aforementioned waste materials have to be treated in the following manner:
- Said waste materials have to be inserted in container duly differentiated from the containers employed for the other types of waste materials;
- Before their removal, they must be subjected to suitable disinfection or sterilization treatments, required and controlled by the Sanitary Director or by the responsible laboratory director;
- After said treatments the containers of the treated waste materials have to be gastight closed and brought to the plants for their final elimination, which, at present, are the incinerators which must present predetermined characteristics.

These procedures practically require considerable troubles and failures which negatively affect the objects that the Legislator would have desired to reach for the safeguard of the public health and/or of the ambience. More in particular:
- The disinfection of the special waste materials in the inside of their containers is commonly carried out, in the best case, by the use of liquid disinfectants.

These methods do not permit the certain "disinfection or sterilization" of said special waste materials, since their section is only determined by a contact and, therefore, it would be necessary to fill up each whole container with the liquid disinfectant. But also in this case, it would be necessary to check up that said sterilization would have been obtained in the waste materials having a certain thickness, or in the biological materials closed in the inside of containers (for instance, blood contained in test tubes closed by plugs).

In the practice the conditions of disinfection or sterilization, established by the legislator are never been reached.
- This circumstance is enhanced by the fact that the legislator requires, in any case, an incineration of the treated special waste materials, as has been afore mentioned. But the incineration treatment would be unnecessary if there is the absolute certitude that the sterilization of the materials would have been duly carried out.
- The presence in Italy of very few incinerators, taken in consideration together with the disposition that requires the incineration of the aforementioned special waste materials, causes the impossibility of a suitable treatment of all the said special hospital waste materials which are produced, thus promoting many unlawful processes as well as an increase without limit of the elimination costs.

The object of the present invention provides to overcome the aforementioned inconveniences by means of a method and apparatus realizing, on whichever type of special waste materials, their certain sterilization, taking in due consideration the objects of the present rules. By the use of the method and process of the present invention, after the treatment, the special hospital waste materials present a bacterial charge equal to zero; therefore that permits to classify and consider the treated materials, from an hygienic point of view, more secure than the solid urban waste materials so that the former ones can be treated as these latter.

The present invention is based on a method of a "dry heat" sterilization applied for a predetermined period of treatment.

A dry heat sterilization, when applied according to the due expedients, produces an unfailing microbicidal action in the innermost parts of materials having a high thickness or in biological materials closed into containers and the like and that in contrast to what takes place by the use of the steam sterilization method (i.e.; by employing autoclaves).

The apparatus comprises a tunnel consisting of a series of a member of modular units so selected as to permit to use an apparatus corresponding to the volume of the waste materials to be treated. Thus the tunnel defines a tubular treatment chamber of a variable capacity open at its ends, where doors are mounted adapted to gastight close said tubular chamber: said doors can be automatically be closed and opened. In the inside of said treatment tubular chamber a continuous conveyor is mounted consisting of an independent unit which can be partially caused to let partially come out from one or the other of the ends of said tunnel.

The conveyor comprises a continuous belt or band made of steel net or the like, which is caused to move with a predetermined and adjustable speed; on said metal belt during each operative cycle are mounted one or more containers of the special waste materials to be treated.

Along the whole treatment chamber heating means are mounted apt to create an atmosphere of dry heat in the inside of said tunnel and which permits the total sterilization of the materials having a bacterical content or which are, however, dangerous. Said heating means act under the control of a probe and are so programmed as to be able to produce the necessary heat, according to the characteristics of the materials to be treated and of the duration of the holding thereof in the tunnel.

One at least of the modular units, that will be preferably mounted at the center of the apparatus, extends upwards with a chimney, at the base of which a precombustion chamber is arranged, in which smoke suctioning means and burners are mounted, so that only sterilized smokes can be discharged.

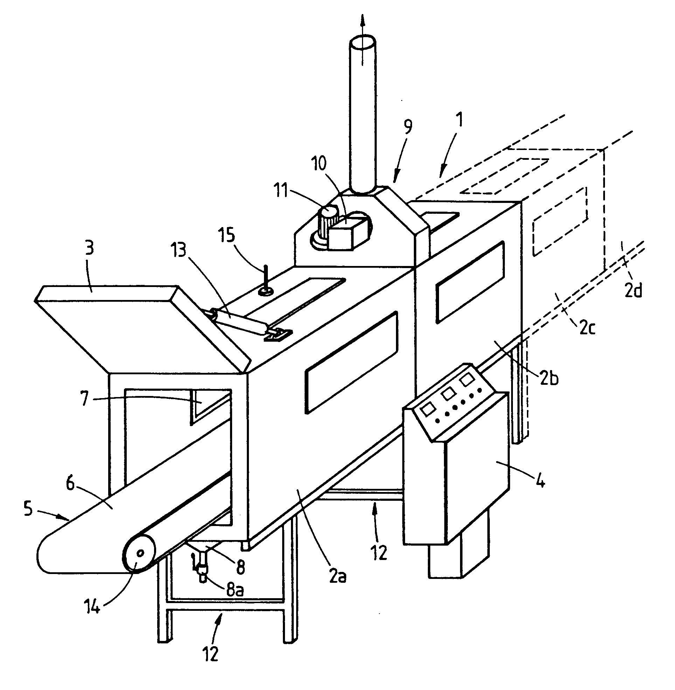

These and other characteristics of this invention will be better understood from the following description of an embodiment of the apparatus reference being made to the accompanying drawing, the sole Figure of which shows a partial perspective side view of a treatment tunnel view that is taken from one of its ends, where the closure door is in open position and where from the open door of said tunnel an end portion of the conveyor has been let come out.

Now referring to the drawing, 1 indicates the treatment tunnel consisting of a series of modular units 2a,2b,.. which are designed to form, in a pre-selected combination, a tunnel 1 of the desired length of its inner treatment chamber, that has a substantially constant cross section.

Each of the modular units 2a,2b,..; is supported by a framework 12.

The tunnel 1 defines in its inside an inner tubular treatment chamber, open at its ends, where doors 3 are mounted which can be caused to be automatically gastight closed or opened by oleodynamic control jacks 13 or the like, according to the duration of the operative cycle.

Along the bottom wall of the treatment chamber a continuous belt conveyor in longitudinably movable manner is mounted the continuous belt or band of which consists of a metal net, made preferably of stainless steel. The belt 6 at the ends of the conveyor 5 passes around two parallel drums 14, one of which is a driving drum.

The conveyor 5 is independent from the tunnel structure so that it can be pushed or pulled out in such a manner that one or the other of the end portions of said conveyor can be caused to come out from one or the other of the ends of the treatment chamber, as the respective door 3 will be opened.

That takes place when one or more containers have to charged upon the conveyor 5, or when one or more containers which contain the now steril waste materials has or have to be removed from the conveyor at the end of the sterilized cycle.

In the inside of the treatment chamber heating means 7 are mounted, as for istance, ultraviolent ray lamps and the like, connected to a feeding source and to adjusting means which comprise control devices mounted on a control board 4 and which act under the control of at least a probe 15. On said board 4 there are also the control devices for programming the motion times of the belt 6 of the conveyor 5 and for the opening and closure of the doors 3.

At least one of the modular units 2a,2b,..., the units 2b, for instance, is made integral with a suction chimney 9, at the base of which a precombustion chamber is obtained, in which means 10 are arranged for the suction of the gas or smoke from the treatment chamber as well as burners 11 provided to purify the suctioned smoke and gases and to discharge them through the chimney 9 in the atmosphere, after having been deprived of any polluting and noxious content.

In the bottom surface of the tunnel 1, underneath the conveyor 5, a longitudinal collectiong channel 8 is arranged for collecting the liquids which may be separated from the treated waste materials and which are discharged through pipe unions 8a, provided with closure means.

## Claims

1. A method for sterilizing dangerous hospital waste materials, collected in special containers and which comprises a dry heat sterilization of said materials in the inside of a tubular room sealingly closed at its ends by openable means, in said treatment room means being provided apt to move continuously forwards said containers from one end of the room towards the other end of said room with a predetermined speed.

2. Apparatus for carrying out the method according to claim 1, characterized by the fact, that it comprises a tunnel (1) consisting of a series of a preselected number of modular units (2a,2b,...) apt in combination to form a tunnel (1) that defines in the inside a tubular treatment chamber of substantially equal cross sections, and having such length to give to said chamber the required volume, said tunnel (1) having open ends, which can be closed gastight by doors (3), the opening and closure of which is controlled by automatic control means (13) oleodynamic jacks, for instance, in the treatment chamber near the bottom wall of the tunnel (1) a continuous belt conveyor is mounted, consisting of an independent motorized assembly, which can be caused to slide along said bottom longitudinally so as to be let come out partially from one or the other of the treatment chamber ends of the tunnel (1), as the respective door (3) opens, the belt (6) consisting of a metal net, preferably, of stainless steel, while in the inside of each units (2a,2b,...) forming the tunnel (1), heating means (7) are mounted, apt to produce dry heat, as, for instance, ultraviolent ray lamps associated with means for adjusting the temperature of the treatment chamber, acting under the control of probes (15), according to the amounts of the materials of the treated and the holding of said materials in the tunnel (1), at least one of said modular units (2a,2b,...) extending upwards with a smoke discharge chimney (9) at the base of which a precombustion chamber is provided, in which devices (10) are mounted for sucking the smoke out of the treatment chamber, as well as burners (11) for the sterilization of said smoke, while in the upper surface of the bottom wall of the treatment chamber a longitudinal channel (8) is arranged provided for collecting the percolate that is then discharged through pipe unions (8a), provided with closure means.

## Patentansprüche

1. Verfahren zur Sterilisierung von gefährlichem Krankenhausabfällen der in speziellen Behältern gesammelt wird, mit einer trockenen Sterilisationsphase unter Wärmezufuhr der genannten Materialen, innerhalb einer rohrförmigen Kammer die an ihren Enden geschloßen und abgedichtet ist, durch Mittel die geöffnet werden können, wobei in dieser Behandlungskammer Mittel vorgesehen sind, die erlauben, die genannten Behälter ständig nach vorne zu bewegen, von einem Ende der Kammer in Richtung des anderen Endes, mit einer vorgegeben Geschwindigkeit.

2. Vorrichtung zur Ausführung des obigen Verfahrens nach Anspruch 1, dadurch gekennzeichnet daß sie ein Tunnel (1), das aus einer Reihe einer vorgegebenen Anzahl von Modulen (2a, 2b,...) besteht, aufweist, wobei die genannten Modulen gemeinsam das Tunnel bilden, das in seinem Inneren eine rohrförmige Behandlungskammer definiert mit im wesentlichen dieselben Querschnittsflächen und mit einer Länge die dieser Kammer das vorgeschriebene Volumen erteilt; wobei dieses Tunnel (1) offene Enden besitzt dei man gasdicht durch Türen (3) schließen kann deren Offnüngs - und Schließbewegung durch automatische Steuerungsmittel (13) gesteuert wird, z.B. durch öldynamische Triebe; wobei in der Behandlungskammer neben der Basisfläche des Tunnels (1) ein endloses Förderband angeordnet ist, bestehend aus einer unabhängigen durch einen Motor angetriebenen Baugruppe die auf dieser Basisfläche entlanggleitet und die zum Teil aus dem einen oder dem anderen Ende der Tunnelbehandlungskammer (1) herausragt wenn die betreffende Tür (3) aufgemacht wird, wobei das Band (6) aus einem metallischen Netz besteht, vorzugsweise aus rostfreiem Stahl, und wobei ferner innerhalb jedes Moduls (2a, 2b, ...) der das Tunnel (1) bildet, Heizmitteln (7) angeordnet sind die "trockene Wärme" liefern wie, z.B., Lampen zur Ausstrahlung von UV-Strahlung die Mitteln zugeordnet sind zum Regeln der Temperatur der Behandlungskammer und die unter der Kontrolle von Sonden (15) stehen, entsprechend der Menge der zu behandelnden Materialen und deren Verweilzeit in dem Tunnel (1), wobei wenigstens ein Modul (2a, 2b,...) sich nach oben erstreckt und eine Rauchhaube bzw. einen Kamin (9) bildet, auf deren/dessen Basis eine Vorverbrennungskammer angeordnet ist innerhalb welcher Vorrichtungen (10) die den Rauch von der Behandlungskammer absaugen, und Brenner (11) zur Sterilisation des Rauchs, montiert sind, während auf der oberen Oberfläche der unteren Behandlungskammerwand ein länglicher Kanal (8) vorgesehen ist zum Sammeln der Perkolationsflüssigkeit die dann innerhalb von Rohranschlüßen (8a) abfliesst, welche Verschlußmittel besitzen.

## Revendications

1. Procédé pour la stérilisation des déchets dangereux des hôpitaux contenus dans des récipients spéciaux, qui comprend une stérilisation à chaleur sêche des décrets sousdits à l'intérieur d'une chambre tubulaire dont les bouts sont fermés hermétiquement par des moyens ouvrants, des moyens étant prévus dans cette chambre de traitement qui déplacent continuellement lesdits récipients d'un bout à l'autre de la chambre à une vitesse prédéterminée.

2. Dispositif pour la réalisation du procédé selon la Revendication 1, caractérisé en ce qu'il comprend un tunnel (1) composé d'une série d'unités modulaires au nombre présélectionné (2a, 2b, ...) dont la combinaison forme un tunnel (1) qui définit à l'intérieur une chambre de traitement tubulaire aux coupes transversales essentiellement égales, et ayant une longueur appropriée pour donner à ladite chambre le volume requis, le tunnel (1) ayant des bouts ouverts qui peuvent être fermés hermétiquement par des portes (3) dont l'ouverture et la fermeture sont commandées par des moyens de commande automatique (13), des vérins oléodynamique par exemple; dans la chambre de traitement près de la paroi inférieure du tunnel (1) est monté un convoyeur à bande continue, composé d'un assemblage mécanique indépendant, qui peut glisser longitudinalement le long de ladite paroi inférieure de façon à sortir partiellement d'un bout ou de l'autre de la chambre de traitement du tunnel (1), quand la porte (3) respective s'ouvre, la bande (6) étant composée d'un filet métallique, en acier inox de préférence, alors qu'à l'intérieur de chaque unité (2a, 2b, ...) formant le tunnel (1) sont montés des moyens de chauffage (7) qui produisent de la chaleur sèche, tels que, par exemple, des lampes ultraviolettes associées à des moyens de réglage de la température de la chambre de traitement, qui agissent sous le contrôle de sensors (15), sur la base de la quantité des déchets à traiter ainsi que de la durée du séjour de ces déchets dans le tunnel (1), au moins une des unités modulaires (2a, 2b, ...) s'étendant vers le haut par une chéminée de décharge de la fumée (9) à la base de laquelle est prévue une chambre de précombustion, où sent montés des dispositifs (10) qui aspirent la fumée de la chambre de traitement, ainsi que des brûleurs (11) qui stérilisent ladite fumée, alors que sur la surface supérieure de la paroi inférieure de la chambre de traitement est prévu un canal longitudinal (8) qui recueillit la percolation qui est successivement déchargé par des raccords union (8a) munis de moyens de fermeture.
